Europäisches Patentamt

European Patent Office

Office européen des brevets

(19)

(11) Veröffentlichungsnummer: **0 053 633**

**A1**

(12) **EUROPÄISCHE PATENTANMELDUNG**

(21) Anmeldenummer: **80107711.6**

(22) Anmeldetag: **08.12.80**

(51) Int. Cl.³: **A 61 F 1/03**

(43) Veröffentlichungstag der Anmeldung:
**16.06.82 Patentblatt 82/24**

(84) Benannte Vertragsstaaten:
**CH FR GB LI**

(71) Anmelder: **Schneider, Wolfgang, Dr.**
**Weddigenufer 12**
**D-4900 Herford(DE)**

(72) Erfinder: **Schneider, Wolfgang, Dr.**
**Weddigenufer 12**
**D-4900 Herford(DE)**

(74) Vertreter: **Thielking, Bodo, Dipl.-Ing. et al,**
**Patentanwälte Bodo Thielking Otto Elbertzhagen**
**Gadderbaumer Strasse 20**
**D-4800 Bielefeld 1(DE)**

(54) Oberarmkopfprothese.

(57) Eine Oberarmkopfprothese besitzt einen durchgehenden Kanal (2) zur Aufnahme der langen Bizepssehne (15), Eine Abdeckung (3) ermöglicht es, die lange Bizepssehne ohne Trennung in den Kanal einzulegen und anschließend den Kanal seitlich zu verschließen.

./...

EP 0 053 633 A1

FIG.4

## Oberarmkopfprothese

Die Erfindung betrifft eine Oberarmkopfprothese mit einem Zapfen zum Einsetzen in den Oberarmknochenschaft und mit einem sich an den Oberarmknochenschaft anschließenden Prothesenschaft sowie einem zumindest im wesentlichen kugeligen Prothesenkopf, der einen leicht geneigt zur Längsachse der Prothese verlaufenden Kanal aufweist.

Bekannte Oberarmkopfprothesen dieser Art (z. B. DE-GM 71 46 082) werden nach der Resektion des natürlichen Oberarmkopfes mit dem Zapfen in den verbleibenden Oberarmknochenschaft eingesetzt und darin fixiert. Vor dem Resezieren des natürlichen Oberarmkopfes und vor dem Einsetzen des Zapfens der Prothese ist es bisher erforderlich und üblich, die lange Bizepssehne, die am Rabenschnabelfortsatz angewachsen ist, zu trennen. Die lange Bizepssehne wird nach dem Einsetzen der Prothese an dem Prothesenkopf befestigt. Dies führt dazu, daß einerseits der Arm nur noch geringfügig belastet werden kann und daß außerdem der Prothesenkopf leicht aus der vorgesehenen Normallage auswandert, weil er nicht mehr von der Bizepssehne in seiner vorgesehenen natürlichen Lage gehalten werden kann.

Der Erfindung liegt die Aufgabe zugrunde, eine Oberarmkopfprothese zu schaffen, welche nicht die geschilderten Nachteile der bekannten Prothese hat und zur Herstellung eines praktisch voll funktionsfähigen Schultergelenks geeignet ist.

Zur Lösung dieser Aufgabe wird nach der Erfindung vorgeschlagen, daß der Kanal durchgehend und zur Aufnahme der Bizepssehne ausgebildet ist, sowie eine außen liegende Abdeckung aufweist.

Das Vorsehen eines Kanals im Prothesenkopf erlaubt den Einsatz der Prothese ohne vorher die bisher notwendige Abtrennung der langen Bizepssehne vornehmen zu müssen. Es ist mit der neuen Prothese möglich, die Bizepssehne unverändert zu lassen und durch den erwähnten Kanal der Prothese zu führen. Die außenliegende Abdeckung des Kanals bildet eine sichere Fixierung für die Bizepssehne, so daß diese nicht aus dem Kanal herausspringen kann.

Bei einer bevorzugten Ausführungsform besteht die Abdeckung aus einem den Kanalquerschnitt vollständig übergreifenden, an dem Prothesenkopf befestigbaren, als separates Teil ausgebildeten Abdeckelement. Eine derartige Ausbildung erlaubt einen besonders einfachen Einsatz des Prothesenkopfes und ein Verschließen des Kanals nach dem Einlegen der Bizepssehne in den vorgesehenen Kanal.

Bei einer bevorzugten Weiterbildung der Erfindung ist das Abdeckelement ein Schieber, der in eine zumindest im wesentlichen parallel zum Kanal verlaufende Führung einschiebbar und in radialer Richtung durch Formschluß mit dem Prothesenkopf unverschiebbar festgelegt ist.

Vorzugsweise weist der Schieber einen Verriegelungsstift zum Festlegen des Schiebers in seiner Einsatzlage auf.

Als besonders vorteilhaft hat sich eine Ausführungsform erwiesen, bei der der Prothesenkopf und der
Schieber mit einer Schwalbenschwanzführung ausgebbildet sind.

Schließlich wird erfindungsgemäß noch vorgeschlagen,
daß der Schieber mit seiner Innenseite die Außenwand des Kanals bildet und mit seiner Außenfläche
die Außenkontur des Prothesenkopfes fortsetzt.

Nachstehend wird eine bevorzugte Ausführungsform
der Erfindung anhand der Zeichnung im einzelnen
beschrieben. Es zeigen:

Figur 1    eine Darstellung der natürlichen Anatomie
           im Bereich des Schultergelenks,

Figur 2    die Darstellung gemäß Figur 1 nach der
           Resektion des natürlichen Oberarmkopfes,

Figur 3    die Darstellung gemäß Figur 2 mit einer
           Prothese, die dem üblichen Stand der
           Technik entspricht,

Figur 4    die Anatomie gemäß Figuren 1 bis 3 mit
           der eingesetzten neuen Prothese,

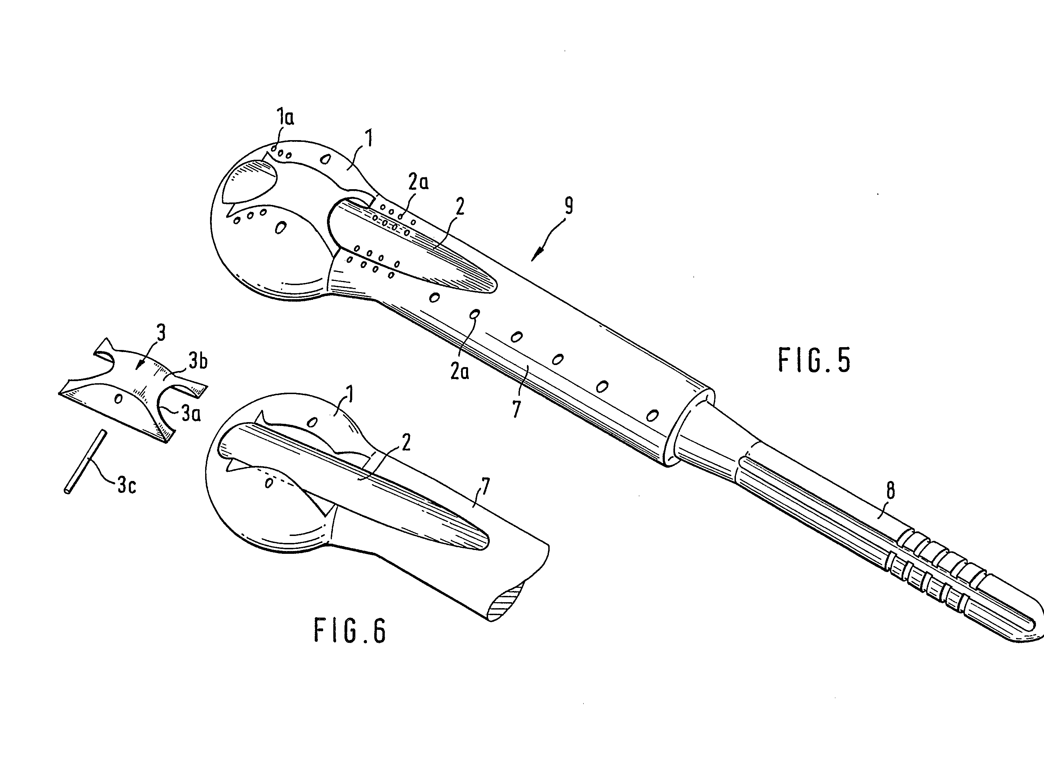

Figur 5    eine perspektivische Darstellung der
           neuen Prothese mit eingesetztem Schieber,

Figur 6 eine perspektivische Darstellung des neuen Prothesenkopfes mit Schieber und Sicherungsstift in Explosionsdarstellung.

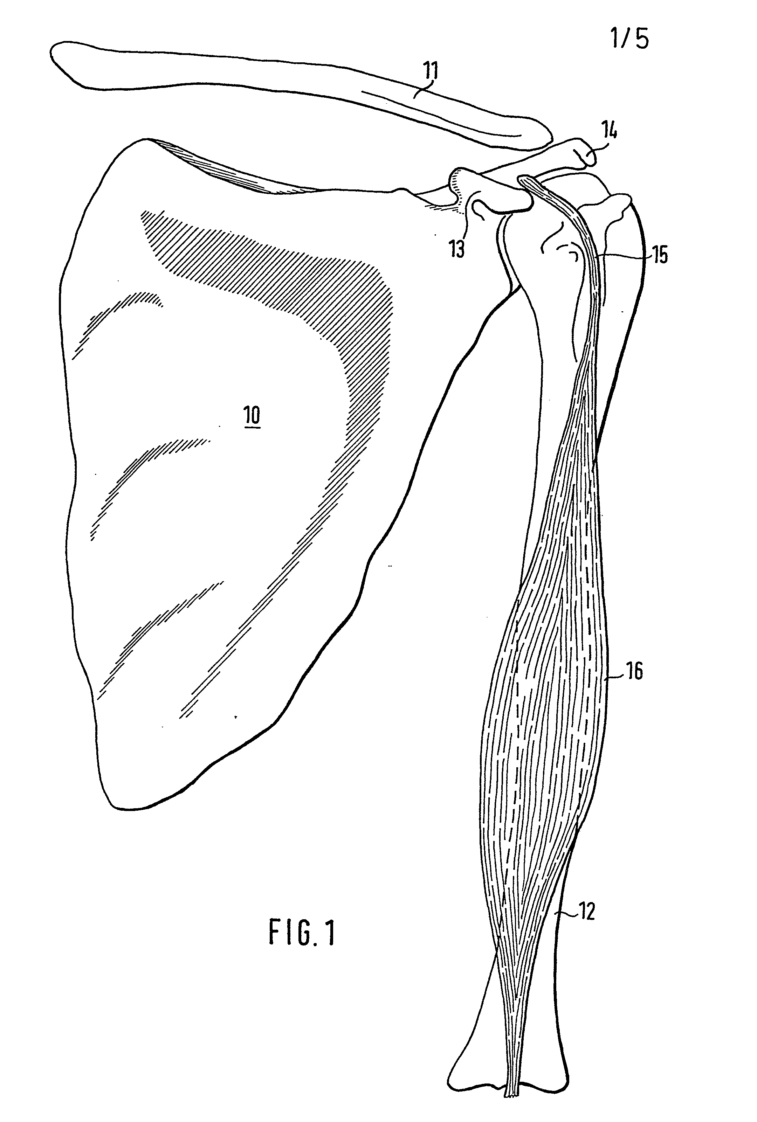

In Figur 1 sind das Schulterblatt 10, das Schlüsselbein 11, der Oberarmkopfknochen 12, der Rabenschnabel 13 und das Acromion 14 sichtbar. Am Acromion ist die Sehne 15 des langen Bizepskopfes 16 angewachsen.

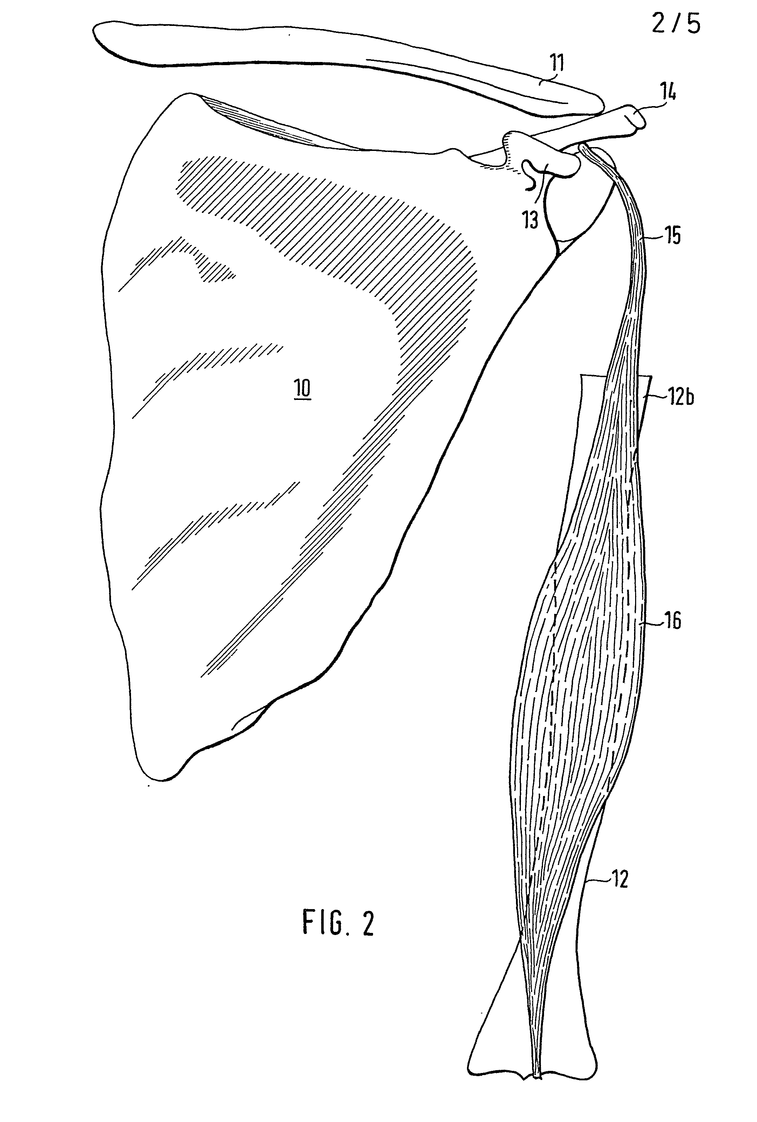

Figur 2 zeigt die Darstellung gemäß Figur 1, bei der der Oberarmkopf 12a bis zum Oberarmschaft 12b abgesägt ist.

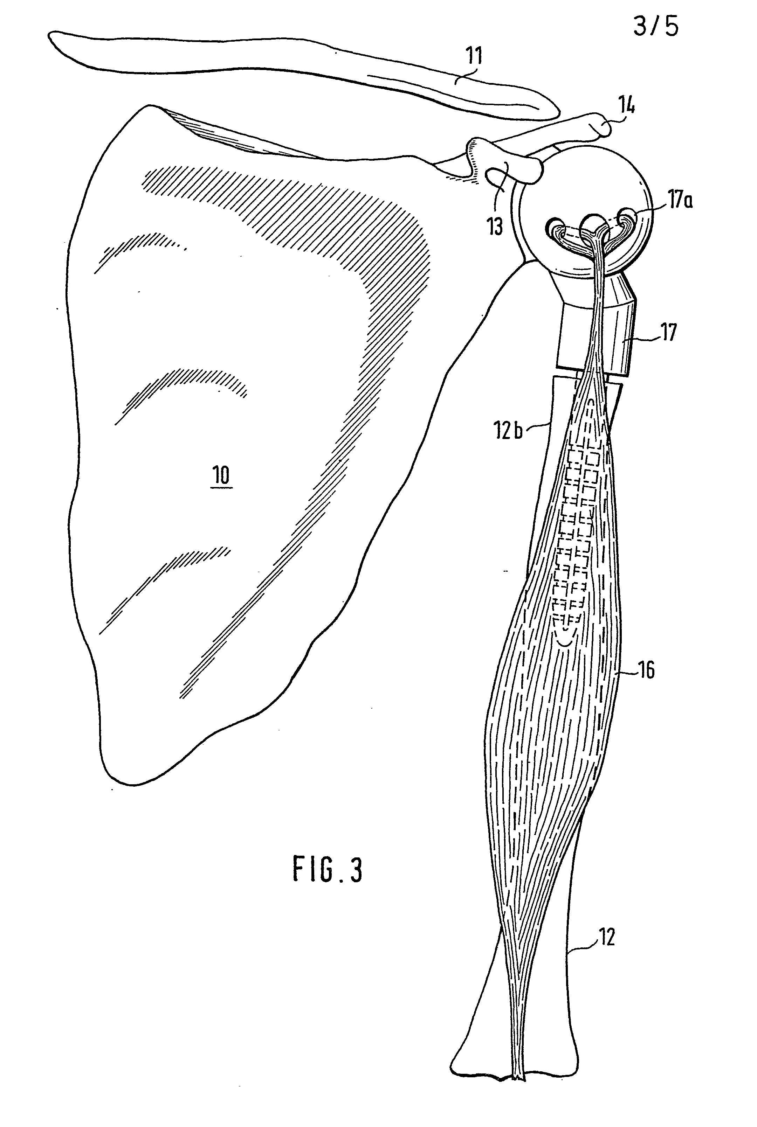

Aus Figur 3 ist der Einbauzustand einer üblichen Oberarmkopfprothese 17 ersichtlich. Die bekannte Oberarmkopfprothese 17 besitzt Durchgangsöffnungen 17a, an denen das vom Acromion abgetrennte Ende der langen Bizepssehne befestigt ist.

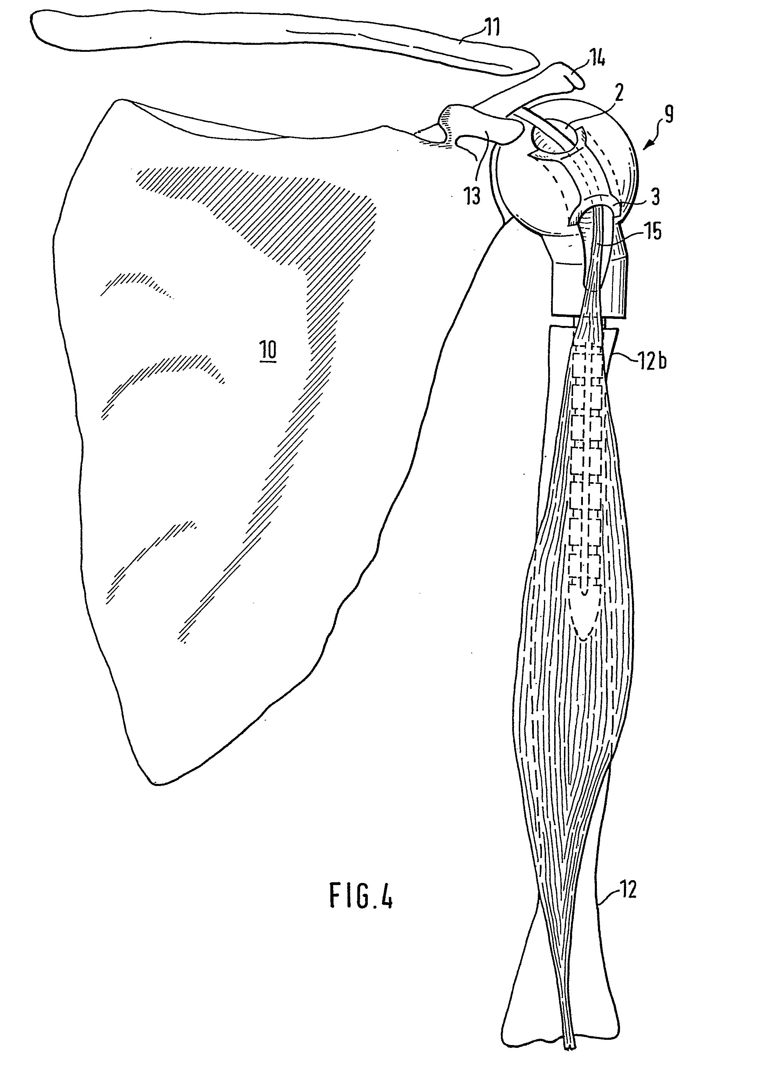

Figur 4 zeigt eine zu Figur 3 analoge Darstellung mit der eingesetzten erfindungsgemäßen Prothese, die insgesamt mit 9 bezeichnet ist. Aus Figur 4 ist ersichtlich, daß die lange Bizepssehne 15 durch den Kanal 2 verläuft und daß der Kanal 2 mittels des Schiebers 3 nach außen abgedeckt ist. Die lange Bizepssehne 15 ist im Kanal 2 frei beweglich.

Die insgesamt mit 9 bezeichnete Oberarmkopfprothese besitzt einen Prothesenkopf 1, einen sich nach unten anschließenden Prothesenschaft 7 und einen zum Eintritt in den Oberarmschaft vorgesehenen Zapfen 8. Durch den Prothesenkopf 1 und einen Teil des sich anschließenden Schaftes 7 erstreckt sich der Kanal 2, der nach außen im Prothesenkopfbereich mittels des Schiebers 3 abgedeckt werden kann. Im Prothesenkopf 1 befindet sich eine etwa parallel zur Längsachse des Kanals 2 verlaufende Schwalbenschwanz-Führungsnut, in die der entsprechend geformte Schieber 3 in Kanallängsrichtung einschiebbar ist. Im eingesetzten Zustand wird der Schieber mittels eines Sicherungsstiftes 3c in der Einsatzlage arretiert. Die Innenseite 3a des Schiebers 3 bildet die Außenwand des Kanals 2. Mit seiner Außenfläche 3b setzt der Schieber 3 die Außenkontur des Prothesenkopfes 1 fort.

Auf dem Prothesenkopf 1 und auf dem Prothesenschaft 7 sind verschiedene Ausnehmungen 1a und 2a vorgesehen, die zur Befestigung der Schulterkapsel dienen.

Patentansprüche:

1. Oberarmkopfprothese mit einem Zapfen zum Einsetzen in den Oberarmknochenschaft und mit einem sich an den Oberarmknochenschaft anschließenden Prothesenschaft sowie einem zumindest im wesentlichen kugeligen Prothesenkopf, der einen leicht geneigt zur Längsachse der Prothese verlaufenden Kanal aufweist,
   dadurch gekennzeichnet,
   daß der Kanal (2) durchgehend und zur Aufnahme der langen Bizepssehne (15) ausgebildet ist sowie eine außenliegende Abdeckung (3) aufweist.

2. Oberarmkopfprothese nach Anspruch 1,
   dadurch gekennzeichnet,
   daß die Abdeckung aus einem den Kanalquerschnitt vollständig übergreifenden, an dem Prothesenkopf (2) befestigbaren, als separates Teil ausgebildeten Abdeckelement besteht.

3. Oberarmkopfprothese nach Ansprüche 1 und 2,
   dadurch gekennzeichnet,
   daß das Abdeckelement ein Schieber (3) ist, der in eine zumindest im wesentlichen parallel zum Kanal (2) verlaufende Führung einschiebbar und in radialer Richtung durch Formschluß mit dem Prothesenkopf (1) unverschiebbar festgelegt ist.

4. Oberarmkopfprothese nach Anspruch 3,
   dadurch gekennzeichnet,
   daß der Schieber (3) einen Verriegelungsstift (4) zum Festlegen des Schiebers in seiner Einsatzlage aufweist.

5. Oberarmkopfprothese nach einem oder mehreren
   der Ansprüche 1 bis 4,
   dadurch gekennzeichnet,
   daß der Prothesenkopf (1) und der Schieber (3)
   mit einer Schwalbenschwanzführung (5,6) ausgebildet sind.

6. Oberarmkopfprothese nach einem oder mehreren
   der Ansprüche 1 bis 5,
   dadurch gekennzeichnet,
   daß der Schieber (3) mit seiner Innenseite (3a)
   die Außenwand des Kanals (2) bildet und mit
   seiner Außenfläche (3b) die Außenkontur des
   Prothesenkopfes (1) fortsetzt.

FIG.1

FIG. 2

FIG.3

FIG.4

FIG.5

FIG.6

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|-----------|---|---|
| | US - A - 3 803 641 (GOLYAKHOVSKY) <br> * Spalte 1, Zeilen 44 bis 46 und Zeilen 61 bis 62 * <br> --- | 1 |
| A | DE - A1 - 2 702 773 (STROOT) <br> * Anspruch 1; Seite 4, Zeilen 5 bis 6 * <br> --- | 1 |
| A | DE - A1 - 2 701 099 (STROOT) <br> * Anspruch 4; Seite 4, Zeile 2 von unten bis Seite 5, Zeile 1 * <br> --- | 1 |
| A | DE - U - 7 237 930 (MATHYS) <br> * Anspruch 3 * <br> --- | 1 |
| D | DE - U - 7 146 082 (MATHYS) <br> * ganzes Dokument * <br> --- | 1 |
| A | US - A - 3 991 425 (MARTIN et al.) <br> * Spalte 2, Zeilen 14 bis 18 * <br> --- | 1 |
| E | DE - A1 - 2 925 145 (SCHNEIDER) <br> * ganzes Dokument * <br> ---- | 1-6 |

**KLASSIFIKATION DER ANMELDUNG (Int. Cl.³)**

A 61 F    1/03

**RECHERCHIERTE SACHGEBIETE (Int. Cl.³)**

A 61 F    1/00

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

X  Der vorliegende Recherchenbericht wurde für alle Patentansprüche erstellt.

| Recherchenort | Abschlußdatum der Recherche | Prüfer |
|---|---|---|
| Berlin | 27-07-1981 | KANAL |

EPA form 1503.1  06.78